# EUROPÄISCHE PATENTANMELDUNG

(11) **EP 2 305 117 A2**
(43) Veröffentlichungstag der Anmeldung: **06.04.2011**
(21) Anmeldenummer: 10158156.9
(22) Anmeldetag: 29.03.2010
(51) Int. Cl.: A61B 5/12

(54) **Verfahren zur Anpassung eines Hörhilfegerätes sowie Hörhilfegeräte-Anpassgerät**

(30) Priorität: 28.08.2009 EP 09168936
(71) Anmelder: Siemens Medical Instruments Pte. Ltd., Singapore 139959 (SG)
(72) Erfinder: Latzel, Matthias, Dr., 91330 Eggolsheim (DE); Giese, Ulrich, Dr., 90762 Fürth (DE); Heuermann, Heike, Dr., 22301 Hamburg (DE)
(74) Vertreter: Maier, Daniel Oliver

(57) **Zusammenfassung**

Es soll aus einer Vielzahl von Daten, die unter Anwendung unterschiedlicher Testmethoden zum Test der auditiven Wahrnehmung einer Person gewonnen wurden, automatisch eine optimierte Hörhilfegeräteparametereinstellung ermittelt werden.

Dieses Ziel wird gemäß der Erfindung dadurch erreicht, dass zunächst durch unterschiedliche Testmethoden Daten bezüglich der auditiven Wahrnehmung (audiologische Daten) des Benutzers gewonnen werden. Die Daten aus den einzelnen Tests können unvollständig, inkonsistent oder untypisch, auch fehlerbehaftet sein. Durch die anschließende Zusammenführung möglichst aller vorliegenden Daten in einer Recheneinheit wird es jedoch möglich, automatisch zu Hörhilfegeräteparametereinstellungen zu gelangen, mittels derer ein damit betriebenes Hörhilfegerät den vorliegenden Hörverlust des betreffenden Benutzers in optimierter Weise ausgleicht.

## Beschreibung

Die Erfindung betrifft ein Verfahren zur automatischen Anpassung einer Übertragungsfunktion eines Hörhilfegerätes für einen bestimmten Frequenzbereich an den individuellen Hörverlust eines Benutzers. Ferner betrifft die Erfindung ein Hörhilfegeräte-Anpassgerät zur Durchführung des Verfahrens.

Unter einer Übertragungsfunktion des Hörhilfegerätes wird insbesondere der Frequenzgang des Hörhilfegerätes, das heißt, die Verstärkung eines Eingangssignals in Abhängigkeit von der Frequenz des Eingangssignals, verstanden. Die Übertragungsfunktion kann außer von der der Frequenz eines Eingangssignals noch von weiteren Signaleigenschaften abhängen, z.B. dem Signalpegel. So ist durch die Übertragungsfunktion auch die signalpegelabhängige Verstärkung (Kompression) bestimmt. Darüber hinaus ist die Übertragungsfunktion auch davon abhängig, ob bestimmte Signalverarbeitungsalgorithmen, z.B. zur Störgeräuschbefreiung, ein- oder ausgeschaltet sind oder ob bei dem Mikrofonsystem eines betreffenden Hörhilfegerätes eine Richtwirkung eingestellt ist. Allgemein wird die Übertragungsfunktion maßgeblich durch den oder die in einer Signalverarbeitungseinheit des Hörhilfegerätes ablaufenden Signalverarbeitungsalgorithmus bzw. Signalverarbeitungsalgorithmen bestimmt, wobei darauf durch die Einstellung von Parametern Einfluss genommen werden kann. Mittels derartiger ParameterEinstellungen ist die Übertragungsfunktion an den individuellen Hörverlust eines Benutzers anpassbar.

Die Anpassung der Übertragungsfunktion eines Hörhilfegerätes an den individuellen Hörverlust des Benutzers erfolgt in der Regel im Dialog zwischen einem Hörhilfegeräteträger und einem Akustiker. Dem Hörhilfegeräteträger werden dabei unterschiedliche Testsignale dargeboten, die er subjektiv wahrnimmt und zu denen er seine Eindrücke dem Akustiker mitteilt. Dieser vergleicht die Wahrnehmung des Hörhilfegeräteträgers mit den Eindrücken Normalhörender auf das jeweilige Testsignal. Aus den unterschiedlichen Empfindungen leitet der Akustiker Hörhilfegeräteparametereinstellungen ab, die in der Regel zu einer verbesserten Anpassung des Hörhilfegerätes an den Hörhilfegeräteträger, insbesondere zu einer individuell optimierten Signalverarbeitung führen. Dieses Vorgehen wird so lange wiederholt, bis der Schwerhörige eine Anzahl an Testsignalen subjektiv ähnlich empfindet wie ein Normalhörender.

Es gibt Situationen, in denen es nicht möglich ist, ein Standard-Audiogramm aufzunehmen, auf dessen Basis die Anpassung erfolgen könnte. Ein Beispiel hierfür sind Entwicklungsländer, in denen es an der erforderlichen audiometrischen Ausrüstung fehlt. Ein weiteres Beispiel sind nicht kooperative Personen, wie kleine Kinder oder bestimmte Personen mit Mehrfach-Behinderungen. Häufig fehlt es auch an einer zur Durchführung einer Reintonaudiometrie erforderlichen, ausreichend ruhigen Umgebung. Es ist daher häufig erforderlich, auf andere Testmethoden zur Gewinnung von Daten bezüglich der auditiven Wahrnehmung einer Person zurückzugreifen.

Neben der Reintonaudiometrie ist eine Vielzahl weiterer Geräte und Testmethoden bekannt, mittels derer man sich einen Eindruck von den Fähigkeiten bezüglich der auditiven Wahrnehmung einer Person verschaffen kann. Darunter fallen einfache Hörtestgeräte, Programme für eine Online-Audiometrie über das Internet, klinische Supra-Schwellen-Tests oder Sprachverständlichkeitstests in Ruhe und in Störlärm. Auch Fragebögen bezüglich typischer Symptome des jeweiligen Hörverlustes können verwendet werden.

Aus der Offenlegungsschrift DE 32 05 685 A1 ist ein Hörgerät mit einem Testtongenerator bekannt, mit dem in einfacher Weise audiologische Daten eines Benutzers gewonnen werden können. Das Hörgerät rechnet die audiologischen Daten selbsttägig in Hörgeräteparametereinstellungen um, durch die das Hörgerät eine Übertragungsfunktion zum Ausgleich des gemessenen Hörverlustes ausführt.

Aus der Druckschrift EP 1 073 314 A1 ist ein Verfahren zur individuellen Anpassung der Signalverarbeitung eines Hörhilfegerätes an einen Benutzer bekannt, bei dem mittels einer Messeinrichtung unterschiedliche auditorische, unwillentliche Körpersignale des Benutzers erfasst und ausgewertet werden zur automatischen Erzeugung hörgerätespezifischer Anpassparametereinstellungen. Zu den unwillentlichen Körpersignalen zählen beispielsweise otoakustische Emissionen (OAE) oder akustisch evozierte Potentiale (AEP).

Nachteilig bei den bekannten Verfahren zur Anpassung eines Hörhilfegerätes an den individuellen Hörverlust eines Benutzers ist, dass diese oft unvollständige, inkonsistente oder fehlerhafte Anpassparametereinstellungen liefern.

Aufgabe der Erfindung ist es daher, aus einer Vielzahl von Daten, die unter Anwendung unterschiedlicher Testmethoden zum Test der auditiven Wahrnehmung einer Person gewonnen werden, zu optimierten Hörhilfegeräteparametereinstellungen zu gelangen.

Diese Aufgabe wird durch ein Verfahren mit den Verfahrensschritten gemäß Patentanspruch 1 gelöst.

Die Grundidee der Erfindung besteht darin, durch unterschiedliche Tests unter Anwendung unterschiedlicher Testmethoden Daten bezüglich der auditiven Wahrnehmung (audiologische Daten) des Benutzers zu gewinnen. In Abhängigkeit von der jeweiligen Testmethode und auch vom Verlauf einzelner Tests eignen sich die gewonnenen Daten mehr oder weniger gut zur automatischen Bestimmung einzelner Teilbereiche der Übertragungsfunktion. Es erfolgt daher eine Gewichtung der gewonnenen Daten dahingehend, dass sie - je nach zugrunde liegender Testmethode - mehr oder weniger Einfluss auf die Bestimmung eines bestimmten Teilbereiches der Übertragungsfunktion haben. Die Gewichtung kann im Extremfall soweit gehen, dass die aus einer bestimmten Testmethode gewonnenen Daten auf einen bestimmten Teilbereich der Übertragungsfunktion keinen Einfluss ausüben (ihr "Gewicht" diesbezüglich "Null" beträgt) oder dass ein bestimmter Teilbereich der Übertragungsfunktion ausschließlich durch die unter Anwendung einer bestimmten Testmethode hervorgegangenen Daten (deren "Gewicht" somit "Eins" beträgt) bestimmt wird. In der Regel werden jedoch die aus mehreren Tests hervorgegangenen Daten einen mehr oder minder großen Einfluss auf die Bestimmung eines bestimmten Teilbereichs der Übertragungsfunktion haben.

Die Grundidee der Erfindung besteht insbesondere darin, durch unterschiedliche Tests unter Anwendung unterschiedlicher Testmethoden jeweils für einen Teilbereich des von dem Hörhilfegerät übertragbaren Frequenzbereiches (Teil-Frequenzbereich) besonders aussagekräftige Daten bezüglich der auditiven Wahrnehmung (audiologische Daten) des Benutzers in diesem Teil-Frequenzbereich zu gewinnen. Durch das Zusammenfügen der für die einzelnen Frequenzbereiche gewonnenen Daten lässt sich dann eine optimierte Übertragungsfunktion des Hörhilfegerätes zum Ausgleich eines individuellen Hörverlustes für den gesamten übertragbaren Frequenzbereich bestimmen. Die aus den unterschiedlichen Tests hervorgehenden Daten werden damit bei der Bestimmung der Übertragungsfunktion in Abhängigkeit der Frequenz unterschiedlich gewichtet. Im Extremfall kann dies sogar dazu führen, dass jeder Test die Übertragungsfunktion für einen bestimmten Teilfrequenzbereich ausschließlich bestimmt.

Unter einem Test im Sinne der Erfindung ist dabei die Anwendung einer bestimmten Testmethode zu verstehen, wobei ein Test auch eine Vielzahl von Einzeltests umfassen kann, beispielsweise die Vorgabe unterschiedlicher Frequenzen und Signalpegel bei der Reintonaudiometrie. Insbesondere kann es sich bei einem Test im Sinne der Erfindung auch um einen iterativen Prozess handeln.

Als Beispiele für Testmethoden zur Gewinnung audiologischer Daten seien insbesondere folgende Methoden genannt, welche unterschiedliche Beurteilungen des Gehörs erlauben:
- Tonaudiometrie in Luftleitung (mittels Kopfhörer oder Lautsprecher) oder Knochenleitung (mittels Knochenleitungshörer) :
   Die Tonaudiometrie dient insbesondere zur Bestimmung der Funktion des Innenohrs und Mittelohrs, der Bestimmung der Hörschwelle und der Unbehaglichkeitsschwelle sowie der Schallleitungsanteile der Schwerhörigkeit.
- Lautheitsskalierung (mittels Kopfhörer oder Lautsprecher):
   Die Lautheitsskalierung dient zur Bestimmung der Funktion des Innenohrs, insbesondere der Bestimmung der Lautheitsfunktion zwischen Hörschwelle und Unbehaglichkeitsschwelle.
- Methoden zur Bestimmung des Pegel-, Frequenz- und Zeitauflösungsvermögens:
   Diese dienen zur Bestimmung der Innenohrfunktion.
- Methoden zur Lokalisation:
   Diese dienen zur Bestimmung des binauralen Abgleichs, insbesondere der audiologischen Funktion des Stammhirns.
- Methoden zur Bestimmung der Sprachverständlichkeit allgemein (in Ruhe und in Störschall):
   Diese dienen zur Betimmung der cortikalen Verarbeitung und der Aufmerksamkeit.
- Spezifische Testmethoden zur Erfassung der Sprachverständlichkeit:
   Aus diesen lassen sich zudem auch Ruhehörschwellen abschätzen, ohne dass eine Tonaudiometrie vorliegt. So kann man zum Beispiel aus dem Verstehen von Zahlwörtern auf die Ruhehörschwelle im Tieftonbereich schließen.

Voraussetzung für die Verwendung oben genannter Verfahren ist die aktive Mitarbeit der Testpersonen, welche teilweise nur eingeschränkt gegeben ist, z.B. bei Kleinkindern, bei Mehrfachbehinderungen, bewusstlosen oder nicht kooperativen Testpersonen. Für diesen Fall gibt es inzwischen eine ganze Reihe physiologischer Testmethoden, die ähnlich wie die verhaltensbasierten Methoden unterschiedliche Bereiche des Gehörs testen. Insbesondere seien hier genannt:
- Mittelohr-Impedanzmessung (Tympanometrie und Stapediusreflex) :
   Diese Testmethode dient der Bestimmung der Mittelohrfunktion, der Abschätzung der Unbehaglichkeitsschwelle sowie des Lautheitsausgleichs und der Schalleitungsanteile der Schwerhörigkeit.
- Messung otoakustischer Emissionen:
   Diese Testmethode dient der Bestimmung der Innenohrfunktion sowie der frequenzspezifischen Funktion der äußeren Haarzellen.
- Messung von Hirnstammpotenzialen / BERA (Brainstem Evoked Response Audiometry):
   Diese Testmethode dient der (frequenzspezifischen) Überprüfung der Hörbahn, der Schätzung der Hörschwelle, der Schalleitungsschwerhörigkeit und Innenohrschwerhörigkeit.
- Messung der Cortex-Potenziale / CERA (Cortical Evoked Response Audiometry):
   Diese Testmethode dient der Bestimmung der frequenzspezifischen Verarbeitung im Cortex sowie von Aufmerksamkeitseffekten.
- Messung ereigniskorrelierte Potentiale (ERP):
   Diese Testmethode dient zur Bestimmung der zentralen Wahrnehmungsverarbeitung, insbesondere des Sprachverstehens.

In Abhängigkeit von der jeweils diagnostizierten Funktionsstörung des auditorischen Systems sind unterschiedliche Hörgeräteeinstellungen notwendig, um bestimmte Teilbereiche der Übertragungsfunktion des Hörhilfegerätes zu optimieren und damit einen optimalen Ausgleich des Hörverlusts zu erzielen. So haben die Anteile von Mittelohr und Innenohrschäden am Gesamthörverlust einen direkten Einfluss auf die frequenzspezifische Verstärkungskurve, während die Lautheitsfunktion bzw. die Funktion der äußeren Haarzellen die Dynamikkompression des Hörhilfegeräts bestimmen. Das Auflösungsvermögen im Innenohr sowie die Funktionen des zentralen Gehörs (Lokalisation, Sprachverstehen, bzw. CERA und ERP) wiederum können durch spezifische zusätzliche Algorithmen im Hörhilfegerät wie Störschallunterdrückung oder sprachsensitive Verarbeitung verbessert werden. Eine möglichst genaue Untersuchung dieser verschiedenen Stadien der auditorischen Verarbeitung ist also eindeutig vorteilhaft.

Gemäß der Erfindung erfolgt eine Festlegung, welchen Einfluss die mittels einer bestimmten Testmethode gewonnen Daten, also die aus einem bestimmten Test hervorgegangenen Daten, auf die Bestimmung eines bestimmten Teilbereichs der Übertragungsfunktion haben sollen. Es erfolgt demnach eine Festlegung, mit welchem "Gewicht" die Daten in die Berechnung der Übertragungsfunktion und insbesondere einen bestimmten Teilbereich der Übertragungsfunktion eingehen. So werden beispielsweise zur Bestimmung des Teilbereiches der Übertragungsfunktion, der die Verstärkung eines in das Hörhilfegerät eingehenden Eingangssignals in Abhängigkeit der Signalfrequenz festlegt, zu den einzelnen Testmethoden "Gewichte" für unterschiedliche Signalfrequenzen festgelegt. Beispielsweise erhält ein Sprachverständlichkeitstest in dem für Sprache besonders relevanten Frequenzbereich zwischen 500 Hz und 3 kHz ein besonders hohes Gewicht, in den übrigen Frequenzbereichen ein niedriges Gewicht, das auch "Null" betragen kann.

Die Gewichtung der Testmethoden hinsichtlich ihres Einflusses bei der Bestimmung der Übertragungsfunktion und insbesondere der Bestimmung bestimmter Teilbereiche der Übertragungsfunktion kann vor der Durchführung einer Anpasssitzung in einem Anpassgerät fest hinterlegt sein. Vorzugsweise ist die Gewichtung jedoch adaptiv. So können die aus unterschiedlichen Tests gewonnen Daten beispielsweise einer Plausibilitätsprüfung unterzogen werden und das Gewicht nicht- oder wenig plausibler Daten herabgesetzt werden.

Es gibt Situationen, in denen es nicht möglich ist, die aus einem Test üblicherweise gewonnenen Daten vollständig zu ermitteln, beispielsweise wenn selbst ein Standard-Audiogramm nur noch unvollständig aufgenommen werden kann. Andererseits arbeiten die Anpassalgorithmen moderner Hörhilfegeräte zumeist ausschließlich auf der Basis von Reintonaudiogrammen. Bekannt sind lediglich Transformationsverfahren von BERA-Daten auf Standard-Audiogramm, allerdings ist auch hier keine direkte Kombination aus Datensätzen vorgesehen. Eine Integration weiterer Messergebnisse war bislang nicht vorgesehen.

Die Daten aus den einzelnen Tests können unvollständig, inkonsistent oder untypisch, auch fehlerbehaftet sein. Insbesondere liefern die einzelnen Tests in der Regel nur Daten bezüglich eines Teils des von dem anzupassenden Hörhilfegerät übertragbaren Frequenzbereichs. Durch die erfindungsgemäße Zusammenführung möglichst aller vorliegenden Daten in einer Recheneinheit wird es jedoch möglich, automatisch zu Hörhilfegeräteparametereinstellungen zu gelangen, mittels derer ein damit betriebenes Hörhilfegerät den vorliegenden Hörverlust des betreffenden Benutzers in optimierter Weise ausgleicht.

Insbesondere wird es durch die Zusammenführung der Daten aus den unterschiedlichen Tests möglich, automatisch die Übertragungsfunktion für den gesamten von dem Hörhilfegerät übertragbaren Frequenzbereich festzulegen.

Durch die betreffenden Hörhilfegeräteparameter wird insbesondere die Verstärkung eines in das Hörhilfegerät eingehenden Eingangssignals bei der jeweiligen Frequenz festgelegt. Mittels geeigneter Einstellungen der Hörhilfegeräteparameter, das heißt die Festlegung bestimmter Werte für den jeweiligen Parameter, wird insbesondere die Übertragungsfunktion des betreffenden Hörhilfegerätes bestimmt.

Hörhilfegeräte übertragen in der Regel nicht den gesamten hörbaren Frequenzbereich (0 bis ca. 20 kHz), sondern nur einen bestimmten Frequenzbereich, z.B. 0 bis 8 kHz. Es ist daher zumeist völlig ausreichend, nur für diesen Frequenzbereich audiologische Daten des Benutzers zu ermitteln.

Der in der Recheneinheit verwendete Algorithmus basiert auf wenigstens einer mathematischen Methode, durch die aus mehreren Datensätzen mit punktuellen und/oder unvollständigen und/oder inkonsistenten und/oder untypischen und/oder fehlerbehafteten Daten ein geeigneter Datensatz ermittelt wird. Zu den verwendbaren mathematischen Methoden gehören die Verwendung von Nachschlagetabellen (look-up tables), Methoden der Clusterbildung (data clustering), Methoden der Faktoranalyse (factor analysis) etc. Unvollständige Daten, das heißt Bereiche innerhalb des relevanten Frequenzbereiches, für die keine Daten bzw. die Daten nicht in der erforderlichen Dichte vorliegen, können aus den vorhandenen Daten durch Extrapolation und/oder Interpolation ermittelt werden. Der vorliegende Algorithmus greift dabei vorteilhaft zur Berechnung von Hörhilfegeräteparametereinstellungen auf mehrere der genannten Methoden zurück. Durch den verwendeten Algorithmus werden vorzugsweise alle vorliegenden audiologischen Daten des Benutzers berücksichtigt. Auch die Verwendung eines neuronalen Netzes und/oder von Fuzzy-Logik können vorteilhaft zur Berechnung der Hörhilfegeräteparametereinstellungen dienen.

Die automatische Gewinnung von Hörhilfegeräteparametereinstellungen gemäß dem vorliegenden Algorithmus erfolgt vorzugsweise in mehreren Stufen, wobei unterschiedliche Vorgehensweisen möglich sind.

Bei einer ersten Vorgehensweise wird aus den einzelnen Datensätzen mit audiologischen Daten zunächst ein vollständiger Datensatz mit audiologischen Daten ermittelt, aus dem dann im zweiten Schritt die gewünschte Übertragungsfunktion für ein Hörhilfegerät mit den entsprechenden Parametereinstellungen zum Ausgleich des individuellen Hörverlustes abgeleitet wird. Bei dem vollständigen Datensatz liegen die audiologischen Daten vollständig, das heißt lückenlos in der erforderlichen Form für den maßgeblichen, von dem Hörhilfegerät übertragbaren Frequenzbereich vor. Dabei legt die Hörhilfegeräte-Übertragungsfunktion den Frequenzgang des betreffenden Hörhilfegerätes und damit die Verstärkung eines Einganssignals in Abhängigkeit von der Signalfrequenz fest. Aus dem gewünschten Frequenzgang können dann die erforderlichen Hörhilfegeräteparametereinstellungen zum Erreichen des gewünschten Frequenzganges ermittelt werden.

Bei einer zweiten Vorgehensweise werden aus einzelnen Datensätzen mit audiologischen Daten direkt einzelne, in der Regel unvollständige und/oder bruchstückhafte und/oder inkonsistente Hörhilfegeräte-Übertragungsfunktionen (Teil-Übertragungsfunktionen) ermittelt, die auf einen Teilbereich des relevanten Frequenzbereichs beschränkt sein können. Anschließend wird aus den einzelnen Übertragungsfunktionen gemäß dem erfindungsgemäßen Algorithmus eine vollständige, das heißt sich über den gesamten relevanten Frequenzbereich erstreckende Hörhilfegeräte-Übertragungsfunktion ermittelt, aus der dann die zum Erreichen dieser Übertragungsfunktion erforderlichen Hörhilfegeräteparametereinstellungen abgeleitet werden.

Vorteilhaft liegt gemäß einer bevorzugten Ausführungsform der Erfindung eine Anzahl vorbestimmter, insbesondere den gesamten relevanten Frequenzbereich abdeckender Datensätze, die den Verlauf typischer Hörverluste widerspiegeln (z.B. typische Audiogramme) und/oder typische Hörhilfegeräte-Übertragungsfunktionen (gain settings) bestimmen, in der Recheneinheit vor, wobei durch den verwendeten Algorithmus jeweils der am besten passende vorbestimmte Datensatz ausgewählt wird.

Der Algorithmus zur Gewinnung von Hörhilfegeräteparametereinstellungen kann auf einem speziellen Hörhilfegeräte-Anpass-Computer implementiert sein. Vorteilhaft ist der Algorithmus jedoch auf einem handhaltbaren Gerät, wie einer Hörhilfegeräte-Fernbedienung, einem Mobiltelefon oder einem persönlichen Minicomputer (PDA) implementiert. Die Dateneingabe erfolgt dabei vorzugsweise manuell. Es kommt jedoch auch eine maschinelle, drahtgebundene oder drahtlose Dateneingabe in Frage, zum Beispiel von Daten, die auf Messungen otoakustischer Emissionen (OAE) oder akustisch evozierter Potentiale (AEP) beruhen.

Das handhaltbare Gerät kann auch gleichzeitig als Programmier-Interface zum Hörhilfegerät zur drahtlosen oder drahtgebundenen Programmierung dienen. Alternativ kann das handhaltbare Gerät auch nur die Parameter-Werte aus einem Satz allgemeiner Hörhilfegerätparametereinstellungen ermitteln, insbesondere unter Verwendung einer Nachschlagetabelle.

Die Erfindung wird nachfolgend anhand von Ausführungsbeispielen näher erläutert. Dabei zeigen:
Figur 1 ein Ablaufdiagramm für ein Verfahren gemäß der Erfindung und
Figur 2 die Gewinnung einer Verstärkungskennlinie für ein Hörhilfegerät auf Basis von Hörtests, die unter Anwendung von vier unterschiedlichen Testmethoden erfolgen.

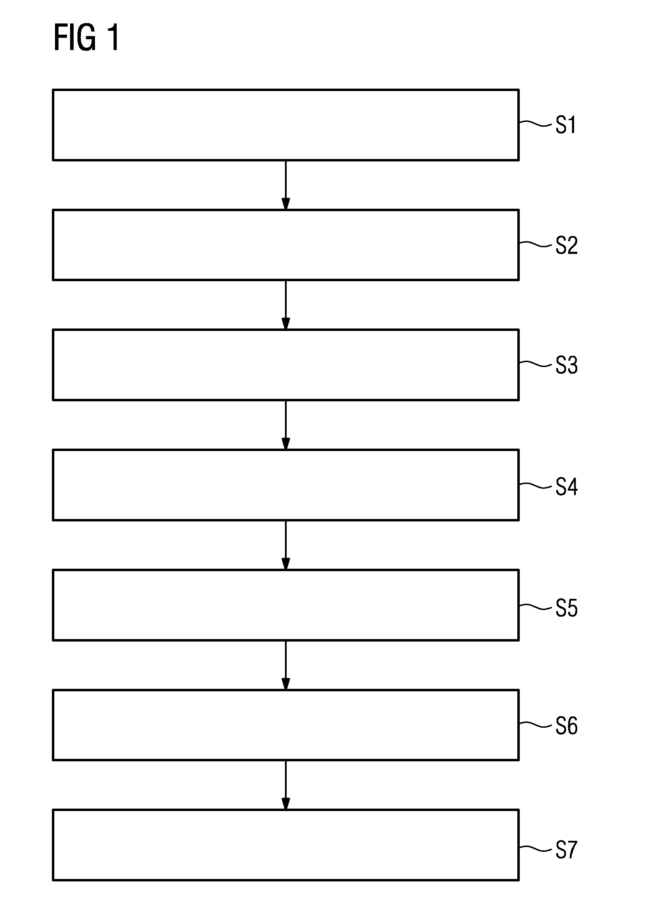

Gemäß einem beispielhaften erfindungsgemäßen Ablaufdiagramm nach Figur 1 erfolgt in einem ersten Verfahrensschritt S1 zunächst wenigstens ein Hörtest unter Anwendung einer objektiven Testmethode (objektiver Hörtest), z.B. Impedanzmessung, Messung otoakustischer Emissionen, BERA- oder CERA-Messungen, Sprachaudiometrie etc.

Anschließend erfolgt in einem zweiten Verfahrensschritt S2 eine Transformation der aus dem objektiven Hörtest gewonnenen Daten in Äquivalente der Standard-Audiometrie. Je nach Art der zugrunde liegenden Messung werden durch den verwendeten Algorithmus zur Datenverarbeitung vorteilhaft unterschiedliche Transformationskurven herangezogen. Es ist davon auszugehen, dass sich nicht immer ein exakter Tonaudiogrammwert zu einer gegebenen Frequenz ermitteln lässt, sondern eher ein Schätzbereich.

Weiterhin erfolgt in einem Verfahrensschritt S3 wenigstens ein Hörtest unter Anwendung einer subjektiven Testmethode (subjektiver Hörtest), z.B. Tonaudiometrie oder Lautheitsskalierung.

Anschließend erfolgt in einem weiteren Verfahrensschritt S4 eine vorzugsweise gewichtete Kombination der im Verfahrensschritt S2 gewonnenen Daten mit den im Verfahrensschritt S3 gewonnenen Daten. Im Idealfall kann dies durch einfache Mittelung von vorzugsweise transformierten Messwerten erfolgen. Bei widersprüchlichen Daten oder fehlenden Werten können vorteilhaft neuronale Netze oder Fuzzy Logic zum Einsatz kommen. Diese neuronalen Netze sollen vor ihrem Einsatz eine Lernphase durchlaufen, in welcher sie auf typische Hörverluste und ihre zugehörigen objektiven und subjektiven Messergebnisse trainiert werden, um später die audiologische Mustererkennung im jeweils vorliegenden Datensatz zu erleichtern. Ein weiteres Lernen basierend auf neuen Messungen kann, muss aber nicht möglich sein. Alternativ kann eine Zuordnung der diversen Messwerte zu einer Hörverlust-Kategorie mittels Clusteranalyse erfolgen. Auch hier besteht die wesentliche Voraussetzung in der zuvor erfolgten Sammlung verschiedener Hörverlusttypen samt ihren zugehörigen Messdaten.

Im nachfolgenden Verfahrensschritt S5 erfolgt die Berechnung einer Verstärkungskurve aus den im Verfahrensschritt 4 gewonnenen kombinierten Daten.

Optional erfolgt in einem weitern Verfahrensschritt S6 wenigstens ein Hörtest zur Ermittlung des Auflösungsvermögen des Gehörs, zur Lokalisation oder zum Sprachverstehen unter Heranziehung von CERA- und/oder BERA-Daten zur Bestimmung von Featureparametern eines einzustellenden Hörhilfegerätes, z.B. bezüglich der Störschallunterdrückung, sprachsensitiver Verarbeitung oder Richtmikrofoneinstellung.

Schließlich erfolgt (ebenfalls optional) in einem Verfahrensschritt S7 die Ermittlung von HörhilfegeräteParametereinstellungen in Abhängigkeit der im Verfahrensschritt S6 ermittelten Daten.

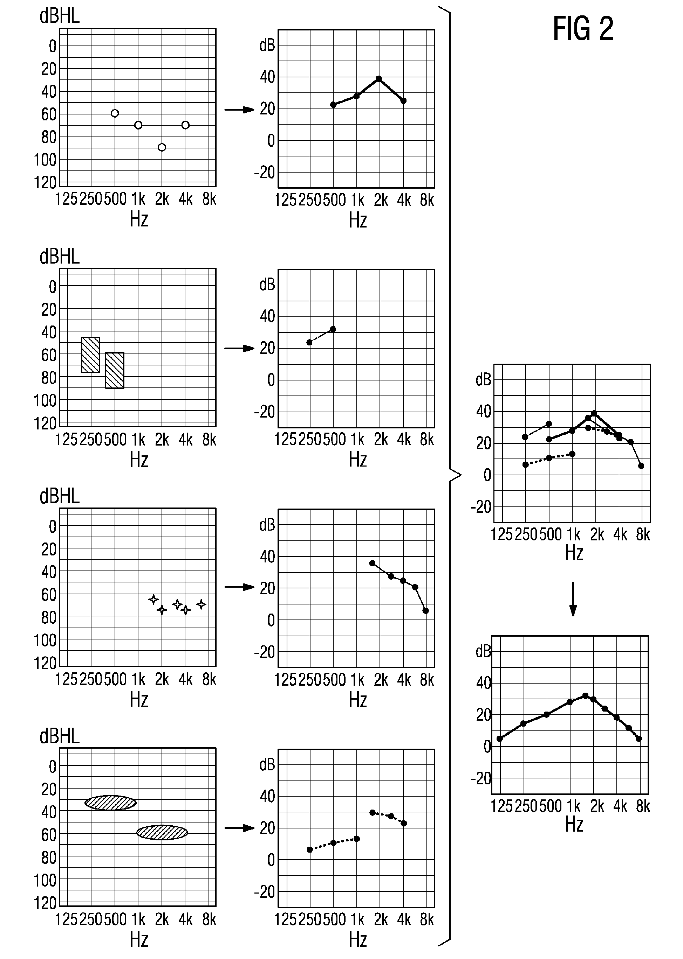

Figur 2 zeigt Ergebnisse von vier verschiedenen Testmethoden, mittels derer Daten über die auditive Wahrnehmung (audiologische Daten) eines Benutzers gewonnen wurden. Es sind dies in der dargestellten Reihenfolge in der linken Spalte der Figur von oben nach unten:
- Das Ergebnis eines Hörtests mit einem Hörtestgerät, bei dem die Hörschwelle bei vier unterschiedlichen Frequenzen ermittelt wurde,
- das Ergebnis eines Sprachverständlichkeitstests,
- das Ergebnis einer Messung otoakustischer Emissionen (OAE),
- das Ergebnis einer Messung von Hirnstammpotentialen.

Die Ergebnisse der Hörtests unter Anwendung der vier verschiedenen Testmethoden sind in Figur 2 jeweils in der linken Spalte grafisch veranschaulicht. Dargestellt ist jeweils der durch den Test ermittelte Hörverlust bei der jeweiligen Signalfrequenz. Vorzugsweise wird das Ergebnis einer jeden Testmethode anschließend automatisch durch den verwendeten Algorithmus in ein Standard-Audiogramm transferiert. Die einzelnen zugrundeliegenden Testmethoden liefern demnach jeweils nur für einen Teilbereich des von dem Hörhilfegerät übertragbaren Frequenzbereichs (Teil-Frequenzbereich) Daten, aus denen bruchstückhaft der Hörverlust der betreffenden Person bei verschiedenen Frequenzen hervorgeht. Demgemäß lassen sich aus den durch die einzelnen Testmethoden gewonnenen Ergebnissen erforderliche Verstärkungswerte für ein Eingangssignal zum Ausgleich des individuellen Hörverlustes der Testperson bestimmen, siehe Schaubilder in der zweiten Spalte von Figur 2. Wie aus den Schaubildern ersichtlich ist, werden die Verstärkungswerte jeweils nur für den Frequenzbereich bestimmt, insbesondere berechnet oder anhand von Nachschlagetabellen ermittelt, für den aus der jeweiligen Testmethode auch Daten vorliegen. Es wird demnach jeweils nur eine Teil-Übertragungsfunktion des Hörhilfegerätes bestimmt.

Im Schaubild in der dritten Spalte sind nun im oberen Diagramm die Ergebnisse aller Schaubilder der zweiten Spalte in einem einzigen Diagramm vereint dargestellt. Wie daraus ersichtlich wird, führen die Testmethoden zu inkonsistenten Ergebnissen. Insbesondere resultieren für einzelne Frequenzen mehrere unterschiedliche Verstärkungswerte. Es werden daher nachfolgend gemäß der Erfindung die aus den unterschiedlichen Testmethoden resultierenden, bruchstückhaften Verstärkungskennlinien zu einer einheitlichen Verstärkungskennlinie, die stetig über den gesamten erforderlichen Frequenzbereich verläuft, kombiniert. Dabei kann auf eine Vielzahl unterschiedlicher mathematischer Methoden - einzeln oder in Kombination - zurückgegriffen werden. Beispiele hierfür sind: Mittelwertbildung, Bildung von Daten-Clustern, Faktoranalyse, Extrapolation etc. Weiterhin kann die Bestimmung einer stetigen Verstärkungskennlinie für den relevanten Frequenzbereich auch unter Anwendung neuronaler Netze und/oder von Fuzzy-Logik erfolgen. Auch die Auswahl der am besten geeigneten Verstärkungskennlinie aus einer Anzahl vorgegebener Verstärkungskennlinien ist möglich. Das Ergebnis dieser Berechnung ist im Ausführungsbeispiel im unteren Diagramm in der rechten (dritten) Spalte veranschaulicht.

Eine bevorzugte Ausführungsform der Erfindung sieht eine unterschiedliche Gewichtung der durch verschiedene Testmethoden gewonnenen audiologischen Daten vor. Dabei kann die unterschiedliche Gewichtung automatisch erfolgen, beispielsweise derart, dass die aus einem Sprachtest gewonnenen Daten mit einem höheren Gewicht als Daten aus einem einfachen Hörtest (Messung der Hörschwelle) in die Ermittlung der Hörhilfegeräteparametereinstellung eingehen. Im Einzelfall können unterschiedliche Gewichtungen außerdem durch manuelle Benutzereingaben festgelegt werden. Darüber hinaus ist auch eine automatische Bewertung der vorliegenden Daten möglich. So kann eine Datenreihe mit sehr vielen Messpunkten ein höheres Gewicht als eine Datenreihe mit nur wenigen Messpunkten erhalten. Auch eine Plausibilitätsprüfung kann zu unterschiedlichen Gewichten führen. So kann eine Datenreihe mit vielen nicht plausiblen Messpunkten bezüglich ihrer Gewichtung herabgestuft werden.

Die Erfindung sieht die Implementierung eines Algorithmus in einem Hörhilfegeräte-Anpassgerät vor, der die oben beschriebene Vorgehensweise umsetzt. Dabei handelt es sich vorzugsweise um ein handhaltbares Gerät. In das Hörhilfegeräte-Anpassgerät werden die durch Anwendung unterschiedlicher Testmethoden gewonnenen audiologischen Daten eingegeben. Zusätzlich oder alternativ kann das Hörhilfegeräte-Anpassgerät auch selbst audiologische Daten erzeugen, beispielsweise indem es einen einfachen Hörtest durchführt. Aus den audiologischen Daten generiert das Hörhilfegeräte-Anpassgerät in der beschriebenen Weise automatisch Hörhilfegeräteparametereinstellung und überträgt diese auf ein anzupassendes Hörhilfegerät, so dass das Hörhilfegerät eine Übertragungsfunktion bezüglich eines in das Hörhilfegerät eingehenden Eingangssignals ausübt, durch die der individuelle Hörverlust des Benutzers ausgeglichen wird.

Bezüglich der Art und Anzahl der verwendbaren Testmethoden zur Ermittlung audiologischer Daten unterliegt die Erfindung keinerlei Beschränkungen. Im einfachsten Fall wird nur eine Hörtestmethode verwendet und die am besten zu dem Testergebnis passende Verstärkungskennlinie aus einer Ansammlung vorgegebener Übertragungskennlinien ausgewählt.

## Patentansprüche

1. Verfahren zur automatischen Anpassung einer Übertragungsfunktion eines Hörhilfegerätes an einen individuellen Hörverlust eines Benutzers mit folgenden Schritten:
- Durchführung unterschiedlicher Tests unter Anwendung unterschiedlicher Testmethoden zur Gewinnung von die auditive Wahrnehmung des Benutzers betreffenden Daten,
- Bestimmung der Übertragungsfunktion in Abhängigkeit der in den unterschiedlichen Tests gewonnenen Daten, wobei bei der Bestimmung der Übertragungsfunktion eine unterschiedliche Gewichtung der aus den unterschiedlichen Tests gewonnenen Daten erfolgt.

2. Verfahren nach Anspruch 1, wobei bei der Bestimmung der Übertragungsfunktion eine frequenzabhängige Gewichtung der aus den unterschiedlichen Tests gewonnenen Daten erfolgt.

3. Verfahren nach Anspruch 2, wobei aus unterschiedlichen Tests gewonnene Daten die Übertragungsfunktion jeweils wenigstens im Wesentlichen nur in einem Teilbereich des von dem Hörhilfegerät übertragbaren Frequenzbereichs beeinflussen.

4. Verfahren nach Anspruch 2 oder 3, wobei die Übertragungsfunktion in einem ersten Teilbereich des übertragbaren Frequenzbereichs zumindest maßgeblich nur von den aus einem ersten Test gewonnenen Daten und in einem zweiten Teil des Frequenzbereichs zumindest maßgeblich nur von den aus einem zweiten Test gewonnenen Daten beeinflusst wird.

5. Verfahren nach einem der Ansprüche 2 bis 4, wobei aus den aus den unterschiedlichen Tests hervorgehenden Daten jeweils Teil-Übertragungsfunktionen des Hörhilfegerätes für einen Teil-Frequenzbereich ermittelt werden und die Teil-Übertragungsfunktionen zu der Übertragungsfunktion für den von dem Hörhilfegerät übertragbaren Frequenzbereich kombiniert werden.

6. Verfahren nach einem der Ansprüche 2 bis 4, wobei die aus den unterschiedlichen Tests hervorgehenden Daten zu Daten für den gesamten von dem Hörhilfegerät übertragbaren Frequenzbereich kombiniert werden und die Übertragungsfunktion aus den Daten für den gesamten übertragbaren Frequenzbereich ermittelt wird.

7. Verfahren nach einem der Ansprüche 1 bis 6, wobei zur Ermittlung der Übertragungsfunktion benötigte, jedoch aus den durchgeführten Tests nicht vorhandene Daten aus vorhandenen Daten extrapoliert werden.

8. Verfahren nach einem der Ansprüche 1 bis 7, wobei Inkonsistenzen in den Daten ermittelt und beseitigt werden.

9. Verfahren nach einem der Ansprüche 1 bis 8, wobei die Ermittlung der Übertragungsfunktion aus den in den unterschiedlichen Tests gewonnenen Daten unter Anwendung von Methoden zur Clusterbildung erfolgt.

10. Verfahren nach einem der Ansprüche 1 bis 9, wobei die Ermittlung der Übertragungsfunktion aus den in den unterschiedlichen Tests gewonnenen Daten unter Anwendung von Methoden zur Faktoranalyse erfolgt.

11. Verfahren nach einem der Ansprüche 1 bis 10, wobei die Übertragungsfunktion aus einer Anzahl vorgegebener Übertragungsfunktionen ausgewählt wird.

12. Hörhilfegeräte-Anpassgerät zur Durchführung eines Verfahrens nach einem der Ansprüche 1 bis 11.
